(19) Europäisches Patentamt / European Patent Office / Office européen des brevets

(11) **EP 2 814 575 B1**

(12) **EUROPEAN PATENT SPECIFICATION**

(45) Date of publication and mention of the grant of the patent:
**11.04.2018 Bulletin 2018/15**

(51) Int Cl.:
*A61K 8/73* $^{(2006.01)}$   *A61Q 5/12* $^{(2006.01)}$
*A61K 8/81* $^{(2006.01)}$   *C08F 220/60* $^{(2006.01)}$

(21) Application number: **13705697.4**

(22) Date of filing: **11.02.2013**

(86) International application number:
**PCT/US2013/025531**

(87) International publication number:
**WO 2013/122861 (22.08.2013 Gazette 2013/34)**

(54) **CONDITIONING COMPOSITION ADDITIVE FOR PROVIDING IMMEDIATE AND LONG LASTING BENEFITS TO KERATIN SUBSTRATES**

ZUSAMMENSETZUNGZUZATZ FÜR SOFORTIGE UND ANHALTENDE VORTEILE AUF KERATIN

ADDITIF POUR COMPOSITION REVITALISANTE PERMETTANT DE PROCURER DES BÉNÉFICES IMMÉDIATS ET DE LONGUE DURÉE À DES SUBSTRATS DE KÉRATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.02.2012 US 201261598031 P**

(43) Date of publication of application:
**24.12.2014 Bulletin 2014/52**

(73) Proprietor: **Hercules LLC
Wilmington, DE 19808 (US)**

(72) Inventors:
• **ERAZO-MAJEWICZ, Paquita
Landenberg, PA 19350 (US)**
• **KROON, Gijsbert
3371 BW Hardinxveld
Giessendam (NL)**
• **NAOULI, Nabil
Wilmington, DE 19808 (US)**
• **NUUTINEN, Tuttu, Maria
NL-2614 TC Delft (NL)**
• **SIEVERLING, Nathalie
47051 Duisburg (DE)**

(74) Representative: **Bülle, Jan et al
Kutzenberger Wolff & Partner
Theodor-Heuss-Ring 23
50668 Köln (DE)**

(56) References cited:
EP-A1- 1 064 927   EP-B1- 1 021 471
WO-A1-94/01076   WO-A1-03/101410
DE-A1- 3 929 973   DE-A1-102004 045 253
US-A1- 2012 076 747   US-B1- 6 489 286

**Description**

CROSS REFERENCE TO RELATED APPLICATIONS

BACKGROUND OF THE INVENTION

1. Field of the Disclosed and Claimed Inventive Concepts

The presently disclosed and claimed inventive concept(s) relates to a conditioning composition additive for use on keratin substrates in order to provide immediate and long lasting benefits to the keratin substrate such as conditioning systems for hair and skin. Specifically, the conditioning composition additive comprises a hydrophobically modified poly(acrylamido-N-propyltrimethylammonium chloride) and water. The conditioning composition additives of the presently disclosed and claimed inventive concept(s) can provide excellent detangling properties, increased hydrophobicity and hair color retention through multiple wash cycles. The presently disclosed and claimed inventive concept(s) also relates to a conditioning composition containing the conditioning composition additive and a method of applying the conditioning composition additive.

2. Background and Applicable Aspects of the Presently Disclosed and Claimed Inventive Concept(s)

Undamaged virgin hair is smooth and shiny; its cuticles on the surface of the hair lie down smoothly making the combing easy. The hair surface is also hydrophobic in nature preventing excessive water absorption during washing. When the hair is either mechanically or chemically damaged through bleaching, perming or coloring, the hair surface becomes rough and frizzy, and difficult to detangle and comb. As the hair surface becomes more hydrophilic, the resulting hair fibers swell during washing making the hair even more difficult to comb. There is an increasing demand for hair care products designed to retain the properties of virgin hair and to prevent possible damage. Healthy hair is less damaged during the chemical treatment than already damaged hair.

Current conditioning systems for damaged hair use one or more combinations of cationic surfactants, amphoteric surfactants, silicones, fatty alcohols, polyquaterniums, amino acids, proteins, lipids and humectants. Wet conditioning of damaged hair is accomplished by neutralizing the anionic charge of the hair by positively charged surfactants and polymers and creating a hydrophobic layer from surfactant and polymers. This hydrophobic layer results a reduction of the swelling of the hair fibers by making the hair more hydrophobic and reducing fiction of the hair fibers. An overall result of wet conditioning is improved detangling, manageability and soft feel of the hair. Upon treatment with cleansing systems like shampoo's, 2/1 shampoo's, body washes or shower gels, the combing performance, detangling properties, hydrophobicity of the hair and lubricity are not maintained sufficiently.

Silicones are traditionally used in hair care applications to provide conditioning properties such as ease of combing and detangling, color retention, smoothness and hydrophobicity. Silicones function by forming a film on the hair surface and depending on the nature of the silicone they are either self-deposited or they require additional cationic ingredients for deposition.

Traditional conditioning products, like rinse off and leave on products contain a formulation chassis of cationic amphoteric surfactants (e.g. behentrimoniurn chloride, cetrimonium chloride, stearamidopropyl dimethylamine etc.) in combination with fatty alcohol (e.g. cetyl alcohol, cetearyl alcohol, stearyl alcohol). In addition to this chassis, the formulations typically contain active ingredients like silicones (e.g. amodimethicone, dimethicone) oils, and proteins (hydrolyzed proteins, quaternized proteins, etc.). These traditional conditioning products do not provide prolonged benefits to keratin surfaces upon washing with cleansing products like shampoos.

WO 03/101410 discloses aqueous conditioning shampoo compositions which comprise an anionic detersive surfactant content; from about 0.025 % to about 5 % by weight of an water soluble or dispersible, cationic, non-crosslinked, deposition or conditioning polymer, the conditioning shampoo may additionally comprise dispersed, liquid, droplets of a water insoluble, hair conditioning agent having a volume average particle diameter of from about 5 microns to about 125 microns.

US 2012/0076747 discloses surfactant-based formulations comprising the polyelectrolytes and blends of such polyelectrolytes with non-cellulosic cationic polysaccharide polymers wherein the surfactant is a phosphate ester.

WO 94/01076 relates to the use of aqueous hair conditioners which contain a combination of cellulose-based cationic polymers with zwitterionic polymers.

US 6,489,286 discloses a conditioning shampoo composition comprised of a mixtures of anionic and amphoteric surfactants and optional conditioners.

EP 1 064 927 A 1 discloses a detergent composition comprising a washing base; at least one water-insoluble silicone; and at least one amphoteric polymer containing 1-20 mole % units derived at least one monomer of (meth)acrylate or (meth)acrylamide type having at least one 8-30 C fatty chain.

DE 10 2004 045 253 discloses a cationic diagonally cross linked copolymer obtained by copolymerisation of at least one monomer A, which may be an ester or amide of acrylic acid and methacrylic acid derived from alcohols or amides having at least 12 carbon atoms, monomer B, which may be an ester or amide of acrylic acid and methacrylic acid having a quaternary ammonium compound, at least doubly unsaturated monomer C, which is copolymerisable with monomers A and B, where in the copolymer, the quantity of B and C is 35-99 wt.% and 0.5-10 wt.%, respectively related to total weight amounts of A and B.

EP 1 021 471 B1 discloses polymer compositions and methods for treating keratin in which a cosmetically acceptable medium is used which contains at least about 0.01 % by weight of an ampholyte polymer comprising acrylamidopropyltrimethyl ammonium chloride or methacrylamidopropyltrimethyl ammonium chloride; acrylic acid, methacrylic acid, 2-acrylamido-2-methylpropanol sulfonic acid or 2-methacrylamido-2-methylpropane sulfonic acid; and, optionally, an alkyl (meth)acrylate.

DE 3929973 discloses aqueous hair care preparations containing zwitterion polymerizates and composed essentially of A) monomers containing quaternary ammonium groups of general formula (I): $R^1CH=CR^2$-CO-Z-$(C_nH_{2n})$-$N^{(+)}R^3R^4R^5$ $A^{(-)}$, where $R^1$ and $R^2$ independently stand for hydrogen or a methyl group and $R^3$, $R^4$ and $R^5$ independently stand for alkyl groups with 1 to 4 carbon atoms, Z stands for an NH group or an oxygen atom, n is an integer from 2 to 5 and $A^{(-)}$ is an anion of an organic or inorganic acid, and B) monomer carboxylic acids of general formula (II): $R^6$-CH=$CR^7$-COOH, where $R^6$ and $R^7$ independently stand for hydrogen or methyl groups, possess hair conditioning and hair setting properties.

BRIEF DESCRIPTION OF DRAWINGS

Further embodiments of the presently disclosed and claimed inventive concept(s) can be understood with the appended figures.

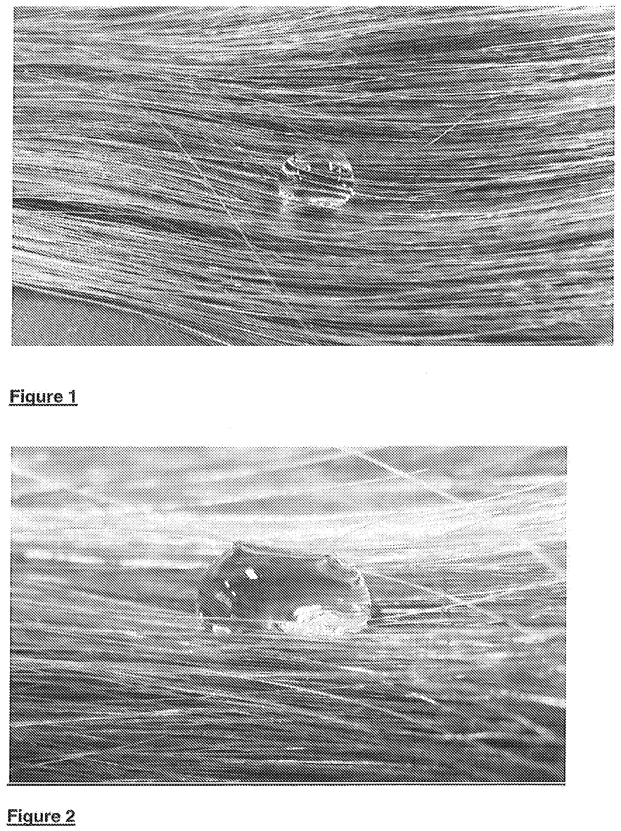

Figure 1 is a photograph of a water droplet on the surface of a bleached Caucasian hair tress treated with a conditioning composition additive of the presently disclosed and claimed inventive concept(s) demonstrating the hydrophobicity imparted to the hair tress by the conditioning composition additive.

Figure 2 is a photograph of a water droplet on the surface of a bleached Caucasian hair tress treated with a conditioning composition additive of the presently disclosed and claimed inventive concept(s) after 5 washes with non-conditioning shampoo demonstrating the hydrophobicity imparted to the hair tress by the conditioning composition additive.

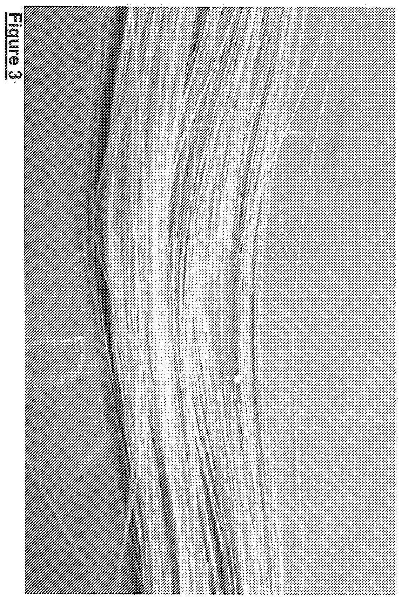

Figure 3 is a photograph of a water droplet on the surface of a bleached Caucasian hair tress treated with a commercially available conditioner.

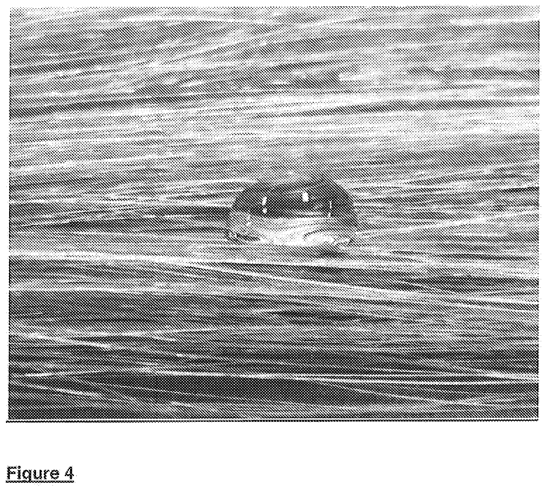

Figure 4 is a photograph of a water droplet on the surface of a bleached Caucasian hair tress treated with a conditioning composition additive in Example 2f as a 1 wt% active in conjunction with cetearyl alcohol at 2.45 wt% and with a cationic surfactant at 0.65 wt% with the balance being water. The tress is being washed 5 times with a silicone-free shampoo.

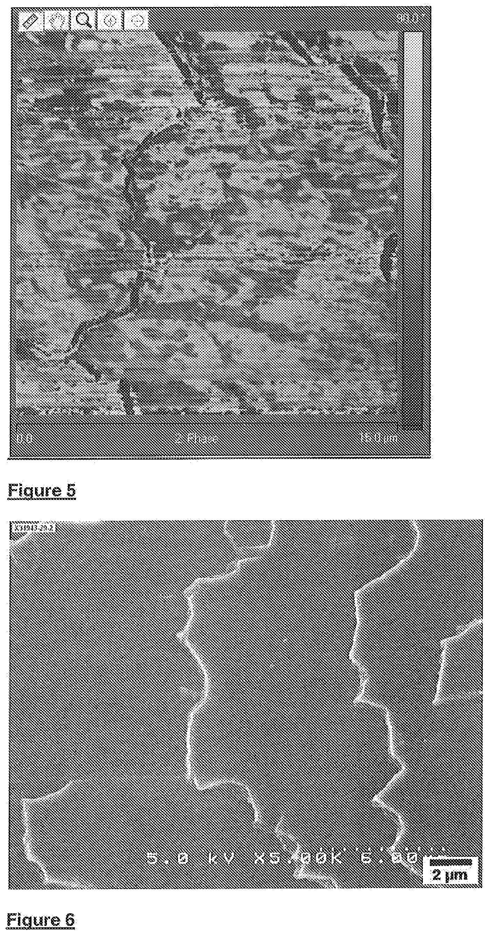

Figure 5 is an AFM image of the surface of a bleached Caucasian hair tress treated with a conditioning composition additive in Example 2f and treated 5 times with following washing cycle: 0.1 grams per gram hair clarifying shampoo containing SLES/CAPB 12/2% and 0.2 grams per gram hair conditioner in Example 2f.

Figure 6 is a SEM image of the surface of a bleached Caucasian hair tress treated with a conditioning composition additive in Example 2f as at 1 wt% active in conjunction with cetearyl alcohol at 2.45 wt% and with a cationic surfactant at 0.65 wt% with the balance being water. After applying the conditioner the tress has been washed 5 times with 0.1 grams per gram hair clarifying shampoo SLES/CAPB 12/2%.

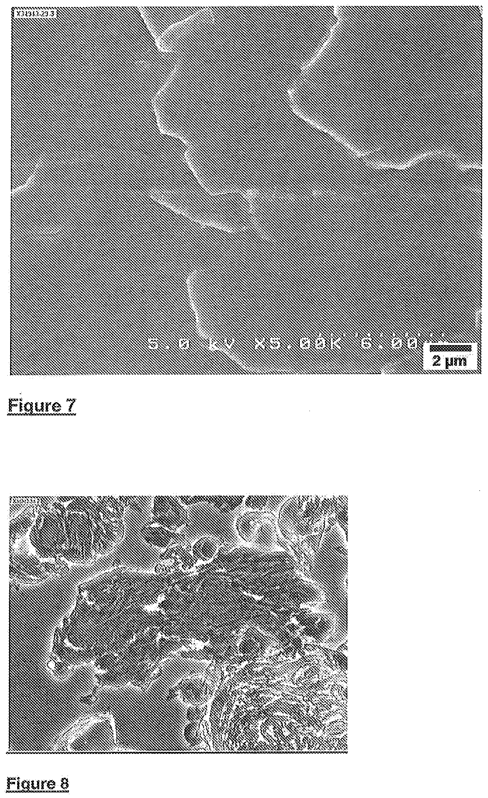

Figure 7 is a SEM image of the surface of a bleached Caucasian hair tress treated with the conditioning composition additive in Example 2f and treated 5 times with following washing cycle: 0.1 grams per gram hair clarifying shampoo containing SLES/CAPB 12/2% and 0.2 grams per gram hair conditioner in example 2f.

Figure 8 is an image of light microscope of an emulsion containing 1 % polymer of Example 2d in combination with 3 % cetearyl alcohol.

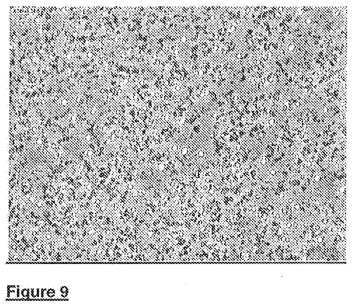

Figure 9 is an image of light microscope of an emulsion containing 1 % polymer of Example 2c in combination with 3 % cetearyl alcohol.

DETAILED DESCRIPTION

Before explaining at least one embodiment of the inventive concept(s) in detail by way of exemplary drawings, experimentation, results, and laboratory procedures, it is to be understood that the inventive concept(s) is not limited in its application to the details of construction and the arrangement of the components set forth in the following description or illustrated in the drawings, experimentation and/or results. The inventive concept(s) is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used in connection with the presently disclosed and claimed inventive concept(s) shall have the meanings that are commonly understood by those of ordinary skill in the art. Further, unless otherwise required by context, singular terms shall include pluralities and plural terms shall include the singular. Generally, nomenclatures utilized in connection with, and techniques of chemistry described herein are those well known and commonly used in the art. Reactions and purification techniques are performed according to manufacturer's specifications or as commonly accomplished in the art or as described herein. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analysis, pharmaceutical preparation, formulation, and delivery, and treatment of patients.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly Indicated to refer to alternatives only or the alternatives are mutually exclusive, although the disclosure supports a definition that refers to only alternatives and "and/or".

Throughout this application, the term "about" is used to indicate that a value includes the inherent variation of error for the device, the method being employed to determine the value, and/or the variation that exists among the study subjects. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results, in addition, the use of the term "at least one of X, Y and Z" will be understood to include alone, Y alone and Z alone, as well as any combination of X, Y and Z.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item o term, such as BB, AAA, MB, BBC, AAABGGCG, CBBAAA, CABABB, and so forth. The skilled artisan will understand that typically there is no limit on the number of items or terms in any combination, unless otherwise apparent from the context.

The presently disclosed and claimed inventive concept(s) relates to a conditioning composition additive for providing immediate and prolonged benefit to a keratin surface. The conditioning composition additive comprises a hydrophobically modified poly(acrylamido-N-propyltrimethylammonium chloride) (polyAPTAC), and water, wherein the hydrophobically modified polyAPTAC is present in an amount of from 0.1 wt% to 20 wt% of the total weight of the conditioning composition additive and has a cationic charge density in the range of 1 to 8 meq/g; and wherein the hydrophobically modified polyAPTAC comprises 1.5% to 15% by weight of a hydrophobic co-monomer selected from the group consisting of alkyl acrylate, alkyl methacrylate, alkyl ethoxylate (meth)acrylate, ethylene glycol behenyl ether methacrylate, and alkyl aryl (meth)acrylate.

It has been surprisingly found that the hydrophobically modified polyAPTAC solutions with relatively low concentrations (< 10 wt.) can provide immediate and prolonged benefits to keratin surfaces in terms of conditioning or color retention after washing with a cleansing product like shampoo. In one non-limiting embodiment, the concentration of the hydrophobically modified polyAPTAC in water may be about 1.0 wt.-%.

Typical concentration of polyAPTAC polymer in solutions as the conditioning composition additive are around 1 wt.-%, which can be adjusted from about 0.1 wt.-% up to about 10 wt.% depending on the viscosity of a final solution. In one non-limiting embodiment, the hydrophobically modified polyAPTAC can be present in an amount of from about 0,25 wt.-% to about 5 wt.-% of the total weight of the conditioning composition additive.

The conditioning composition additive can be applied to hair in a range of about 0.01 - 0.5 gram of hydrophobically modified polyAPTAC solution as an additive per gram of hair. This is similar to the application of conditioners, which can be leave on (typical application dosage < 0.2 gram per gram hair) or rinse off (typical application dosage ≥ 1 gram / gram of hair). The hydrophobically modified polyAPTAC described in this presently disclosed and claimed inventive concept(s) are water soluble.

The hydrophobically modified polyAPTAC in the conditioning composition additive has a cationic charge density of about 1 to about 8 meq/g. In one - non - limiting embodiment, the hydrophobically modified polyAPTAC has a cationic charge density in the range of from about 3 to about 7 meq/g. In another non-limiting embodiment, the hydrophobically modified polyAPTAC has a cationic charge density in the range of about 4 to about 6 meq/g.

The hydrophobically modified polyAPTAC is made by polymerizing acrylamido-N-propyltrimethylammonium chloride (APTAC) with at least one hydrophobic co-monomer. The hydrophobic co-monomers can be added at different amounts to provide various levels of hydrophobicity to the hydrophobically modified polyAPTAC. The hydrophobic co-monomer can be any ethylenically unsaturated monomer. The co- monomers in the presently disclosed and claimed inventive concept(s) are selected from the group consisting of alkyl acrylate, alkyl methacrylate, alkyl ethoxylate (meth)acrylate, ethylene glycol behenyl ether methacrylate and alkyl aryl (meth)acrylate. In one non-limiting embodiment, the alkyl acrylate ca be stearyl acrylate. The hydrophobic co-monomer comprises about 1.5 to about 15 percent by weight based on the total weight of the hydrophobically modified polyAPTAC.

The molecular weight of polymers in this presently disclosed and claimed inventive concepts) can be determined by using standard analytical measurements, such as size exclusion chromatography (SEC), in one non-limiting embodiment, the hydrophobically modified polyAPTAC has a molecular weight in the range of from about 100,000 to about 1,000,000 g/mol. In another non-limiting embodiment, the hydrophobically modified PolyAPTAC has a molecular weight in the range of from about 200,000 to about 500,000 g/mol.

The hydrophobically modified polyAPTAC in the conditioning composition additive can be produced by using any kind of radical polymerization reaction such as solution, emulsion, bulk and precipitation polymerization process. One or more additional monomers can be added in the polymerization. The additional monomers can be selected from the group consisting of cationic, anionic, amphoteric and zwitterionic monomers. For example, but not by way of limitation, the hydrophobically modified polyAPTAC can form copolymers with vinyl monomers, such as vinyl pyrrolidone (VP), acrylate, acrylamide, and vinylalcohol.

The hydrophobically PolyAPTAC can also be combined with any other polymers, such as anionic, cationic, non-ionic, amphoteric or zwitterionic polymers. The hydrophobically modified polyAPTAC in the presently disclosed and claimed inventive concept(s) may be combined with a substantive polymer, for example, but by no way of limitation, a polymer based on a cellulosic or galactomannan backbone. The substantive polymer may be used in combination with the hydrophobically modified polyAPTAC to stabilize the emulsions and acting as a rheology modifier. The substantive polymers can be cationic polysaccharides based on hydroxyethyl cellulose or galactomannan.

In this presently disclosed and claimed inventive concept(s), the substantive polymers can be a hydrophobically modified hydroxyethylcellulose. In one non-limiting embodiment, the hydrophobically modified hydroxyethylcellulose can be in the range of about 0.1 wt % to about 20 wt%. In another non-limiting embodiment, the hydrophobically modified hydroxyethylcellulose can be in the range of about 1 wt% to about 5 wt%.

The hydrophobically modified polyAPTAC can be delivered as a polymer solution or in combination with a fatty alcohol. This conditioning composition additive can show excellent conditioning performance as leave-on and, especially, as rinse-off conditioner. The hydrophobically modified polyAPTAC shows improved conditioning performance over cationic surfactants. In addition, the hydrophobically modified polyAPTAC solutions on their own can provide these benefits, without the need to add cationic surfactants, amphoteric surfactants, fatty alcohols, silicones, proteins, etc....

The presently disclosed and claimed inventive concept(s) also relates to a conditioning composition comprising the conditioning composition additive for providing immediate and prolonged benefit to a keratin surface and a personal care active ingredient.

The conditioning composition additive of the presently disclosed and claimed inventive concept(s) when incorporated into a conditioning composition provides immediate and long lasting performance. Where the conditioning composition is a hair care composition, the benefits which may be extended using the conditioning composition additive include conditioning, detangling, hydrophobicity, improved sensory properties and color lasting after multiple washes with cleansing systems like shampoos, conditioning shampoos, shower gels, etc and with styling applications. Where the conditioning composition is a skin care composition, the benefits which may be extended using the conditioning composition additive include, moisturizing, hydrophobicity or waterproofing, UVA and/or UVB protection.

In the formation of a conditioning composition, the conditioning composition additive is combined with a personal care active ingredient to provide a benefit to the formulation. Due to the cationic nature of the additive, it can be used to deposit other ingredients to the hair and the skin. Examples of other ingredients can include, but are limited to, silicones, emollients, and fragrances, oil-soluble active ingredients such as vitamins, antioxidants and UV-filters.

Examples of the cationic surfactants mentioned in the presently disclosed and claimed inventive concept(s) can be, but are not limited to, cetrimonium chloride, behentrimonium chloride and stearamidopropyl dimethylamine.

The presently disclosed and claimed inventive concept(s) also relates to a method for providing immediate and prolonged benefit to a keratin surface. The method comprises applying a conditioning composition to the keratin surface. The conditioning composition comprises a conditioning composition additive and a personal care active ingredient. The conditioning composition additive and the personal care active ingredient are the same as those described previously.

The following examples illustrate the presently disclosed and claimed inventive concept(s), parts and percentages being by weight, unless otherwise indicated. Each example is provided by way of explanation of the presently disclosed and claimed inventive concept(s), not limitation of the presently disclosed and claimed inventive concept(s).

### EXAMPLES

Procedure for Determining the Cationic Charge Density of Polymers

To determine the cationic charges of polymers, a Mütek charge analyzer was used. The aqueous sample was placed in the measuring cell. Once the PCD was turned on, the piston of the cell oscillated and caused a high flow rate. Any charged material adsorbed to the cell wall was separated from its counter-ions by the flow to create a streaming current. Two electrodes in the cell picked up this current and displayed it. The titration module automatically selected the titrant which was charged oppositely to the sample (cationic sample - anionic titrant) and added it to the sample until 0 mV was reached. As a result the consumption of titrant in ml was indicated on the display as well as the charge demand in meq/g.

Contact Angle Measurements

The immediate and long-lasting hydrophobicity of the polyAPTAC was studied by measuring a contact angle after several washes with a clarifying shampoo. The higher the contact angle the more hydrophobic is the surface. Undamaged virgin brown hair is naturally hydrophobic, but all the chemical treatments such as bleaching reduce the hydrophobicity of the hair. The measurement method is described as follows:

> A portion of the hair tress was stretched on a specially designed plate so that the fibers were suspended together in space to form a "single" surface.
> A droplet of deionized water was delivered from a syringe onto the fiber surface.
> Droplet mass was ~0.008 g.
> Images were collected at intervals of 1s or 10s

Synthesis of PolyAPTAC Homopolymer

Trimethyl ammonium propyl acrylamide chloride (DIMAPA-Q, APTAC) was polymerized in an aqueous media by a discontinuous adiabatic process.

1. PolyAPTAC with Molecular Weight Mw $\approx$ 100,000 g/mol

$320 \pm 10$ kg soft water and $670 \pm 10$ kg APTAC (60%) were added into a vessel and stirred. The pH of the mixture was adjusted with sulphuric acid (50%) to about $5.1 \pm 0.1$. Then, 1.05 kg 2-mercapto ethanol was added at 57 °C. The polymerization was started with addition of 0.30 kg VA-044 (2.2'-Azobis[2(2-imidazolin-2-yl)propane]dihydrochloride) dissolved in water. After reaching the maximum temperature, the vessel was cooled down to < 80 °C. 0.25 kg V-50 (2,2'-Azobis(2-methylpropionamidine)dihydrochloride) dissolved in water was added for burn-out step (about one hour). Finally, the product was cooled down and drummed through a filter.

2. PolyAPTAC with Molecular Weight Mw $\approx$ 200,000 g/mol

$320 \pm 10$ kg soft water and $670 \pm 10$ kg APTAC (60%) were added into a vessel and stirred. The pH of the mixture was adjusted with sulphuric acid (50%) to about $5.1 \pm 0.1$. Then, 0.45 kg 2-mercapto ethanol was added at 57 °C. The polymerization was started with addition of 0.30 kg VA-044 dissolved in water. After reaching the maximum temperature, the vessel is cooled down to < 80 °C. 0.95 kg V-50 dissolved in water was added for burn-out step (about one hour). Finally, the product was cooled down and drummed through a filter.

3. PolyAPTAC with Molecular Weight Mw $\approx$ 300,000 g/mol

579.8 kg soft water and 403.5 kg APTAC (60%) were added into a vessel and stirred. The pH of the mixture was adjusted with 4.3 kg sulphuric acid (50%) to about 5.3-5.4. Then, 0.05 kg 2-mercapto ethanol was added at $\leq$ 75

°C. The polymerization was started with addition of 0.92 kg sodium persulfate dissolved in 2.48 kg water. The reaction was conducted for 1 hour. Then the solution was cooled down. At 70 °C, 0.94 kg sodium persulfate dissolved in 2.48 kg water was added. 10.0 kg water was added for cleaning pipes and tubes. Finally, the product was cooled down and drummed through a filter.

4. PolyAPTAC with Molecular Weight Mw ≈ 500,000 g/mol

591.8 kg soft water and 391.4 kg APTAC (60%) were added into a vessel and stirred. The pH of the mixture was adjusted with 3.657 kg sulphuric acid (50%) to 5.3-5.4. Then, 0.07 kg 2-mercapto ethanol was added at ≤ 75 °C. The polymerization was started with addition of 0.9 kg sodium persulfate dissolved in 2.48 kg water. The reaction was conducted for 1 hour. At 70 °C, 0.9 kg sodium persulfate dissolved in 2.48 kg water was added. 10.0 kg water was added for cleaning pipes and tubes. Finally, the product was cooled down and drummed through a filter.

5. PolyAPTAC with Molecular Weight Mw ≈ 1,000,000 g/mol

592.5 kg soft water and 387.8 kg APTAC (60%) were added into a vessel and stirred. The pH of the mixture was adjusted with 4.63 kg sulphuric acid (50%) to 5.1±0.2. Then, 4.92 kg water was used for washing the pipes and tubes. At 72 °C, the polymerization was started with addition of 0.2 kg sodium persulfate dissolved in 2.46 kg water. 4.92 kg water was used to washing the pipes and tubes. The reaction was conducted for 1 hour. The vessel was cooled down to below 80 °C. 0.24 kg V-50 dissolved in 2.46 kg water was added. 4.92 kg water was used to cleaning the pipes and tubes. Finally, the product was cooled down and drummed through a filter.

Synthesis of Hydriphobically Modified PolyAPTAC

The monomers and co-monomers were polymerized in an aqueous media by a discontinuous process. The polymerization was an adiabatic reaction.

1. PolyAPTAC Modified with Stearyl Acrylate (Mw = 500,000)

295.2 kg soft water, 664.3 kg APTAC (60%) and 13.7 kg pre-emulsion containing 11% cetyl trimethylammonium bromide (CTAB), 44% water and 44% stearyl acrylate were sucked into a vessel Then 7.30 kg sulphuric acid (50%) was added to adjust the pH at 5.1. At 60 °C, 0.15 kg 2-mercapto ethanol was added into the vessel. The polymerization was started with 0.30 kg VA-044 dissolved in 2.70 kg water. After reaching the maximum temperature, 1.0 kg V50 dissolved in 5.4 kg water was added. 10 kg water was used for cleaning the pipes and tubes. Finally, the product was drummed through a filter. The active solid was about 40 wt% and the viscosity was about 7,700 cps. The polymer obtained contained about 1.5% stearyl acrylate.

2. PolyAPTAC modified with stearyl acrylate (Mw ≈ 1,000,000)

295.2 kg soft water, 664.3 kg APTAC (60%) and 13,7 kg pre-emulsion containing 11 % cetyl trimethylammonium bromide (CTAB), 44% water and 44% stearyl acrylate were sucked into a vessel. Then 7.30 kg sulphuric acid (50%) was added to adjust the pH at 5.1. At 72°C, the polymerisation was started with 0.2 kg sodium persulfate dissolved in 2.46 kg water. 4.92 kg water was used to washing the pipes and tubes. The reaction was conducted for 1 hour. The vessel was cooled down to below 80 °C. 0.24 kg V-50 dissolved in 2.46 kg water was added. 4.92 kg water was used to cleaning the pipes and tubes. Finally, the product was cooled down and drummed through a filter. The polymer obtained contained about 1.5% stearyl acrylate.

3. PolyAPTAC Modified with PEG-18 Behenyl Ether Methacrylate (BEM) (Mw ≈ 500,000)

328.0 kg soft water, 633.3 kg APTAC (60%) and 20 kg polyethylene glycol) behenyl ether methacrylate (50%) were sucked into a vessel. Then 4.10 kg sulphuric acid (50%) was added to adjust the pH at 5.0. 5.0 kg water was used to clean the pipes and tubes. At 60 °C, 0.15 kg 2-mercapto ethanol was added into the vessel. The polymerization was started with 0.30 kg VA-044 dissolved in 2.70 kg water and 2.70 g. After reaching the maximum temperature, 1.0 kg V-50 dissolved in 5.4 kg water was added. Finally, the product was drummed through a filter. The active solid was about 40 wt% and the viscosity was about 13,000 cps. The polymer obtained contained about 1.5% BEM.

Example 1 Comparative

As benchmarks two commercial rinse off conditioners were tested as applied and rinsed off and after washing with a simplified shampoo formulation (12 wt% sodium lauryl ether (2EO) sulfate, 2 wt% cocamidopropyl betaine). The products were applied as 0.2 gram of rinse off conditioner per gram of hair. The hair used was damaged by treating virgin brown hair by bleaching for 1 hour. This hair was used for all further testing. The shampoo was applied as 0.1 gram per gram of hair and then rinsed.

**Table 1**

| Example | | Wet comb energy (gl-mm) after rinse-off | Wet comb energy (gf-mm) after 3x shampoo wash |
|---|---|---|---|
| 1a (Comp.) | Gliss Kur Hair repair Total repair | 2653 | 12397 |
| 1b (Comp.) | Dove Therapy Intense™ care conditioner rinse | 1770 | 112,952 |

The combing measurement was done on a texture analyzer without manually detangling the hair. The energy needed to comb the tress was listed as gf-mm. Example 1a contained a cationic polymer in combination with cationic surfactants. Example 1b contained only cationic surfactants. From Table 1 above, it is evident that both of the examples have good initial conditioning properties, but it is also evident that cationic surfactants don't provide long-lasting conditioning.

Example 2 PolyAPTAC Homopolymer (not claimed)

The properties of polyAPTAC homopolymers are presented in Table 2:

**Table 2**

| Example | Actives [wt%] | Mw [g/mol] | Polymer chemistry |
|---|---|---|---|
| 2a | 40 | 100,000 | polyAPTAC |
| 2b* | 40 | 100,000 | polyAPTAC$_{98wt\%}$-$co$-AA$_{2wt\%}$ |
| 2c | 40 | 200,000 | polyAPTAC |
| 2d | 24 | 300,000 | polyAPTAC |
| 2e | 24 | 1,000,000 | polyAPTAC |
| 2f | 24 | 500,000 | polyAPTAC |
| 2b* - a copolymer of 98% APTAC and 2% AA (Acrylic acid) | | | |

The polyAPTAC homopolymers were tested as 1 wt% in 2.45% fatty alcohol and 0.65% cationic surfactants. A reference having 2.45% fatty alcohol and 0.65% cationic surfactant was used. The conditioner was applied on damaged hair (0.2 grams per gram of bleached hair, 1 hour bleached) and wet comb energies were measured with Instron after the rinse with water and after 1, 3 and 5 times of washing with 0.1 grams per gram hair shampoo (shampoo: 12/2 SLES/CAPB). The test results are provided in Table 3.

**Table 3**

| Example | Wet Comb Energy rinse-off conditioner (gf-mm) | Wet Comb Energy 1 wash (gf-mm) | Wet Comb Energy 3 washes (gf-mm) | Wet Comb Energy 5 washes (gf-mm) |
|---|---|---|---|---|
| 2a | 3873 | 6049 | 13,418 | 17,137 |
| 2b | 4468 | 4732 | 16,121 | 16,762 |
| 2c | 4257 | 5699 | 14,951 | 16,134 |
| 2d | 5014 | 3930 | 8596 | 11,720 |

(continued)

| Example | Wet Comb Energy rinse-off conditioner (gf-mm) | Wet Comb Energy 1 wash (gf-mm) | Wet Comb Energy 3 washes (gf-mm) | Wet Comb Energy 5 washes (gf-mm) |
|---|---|---|---|---|
| 2e | 4116 | 3542 | 7649 | 11,541 |
| 2f | 4045 | 4163 | 9832 | 12,493 |
| Reference | 2016 | 16,294 | 21,078 | 20,305 |

From the above results presented in Table 3, it is evident that a polyAPTAC homopolymer with higher Mw (see Example 2e) provides better performance. Yet the differences between different polymers are small and the comb energy results with low Mw (100,000) are adequate and all the combinations are performing better than the reference.

Example 2f was polyAPTAC with a cationic charge of 4.8 meq/g and Mw 500,000 Dalton. Even at very low dosages it is resulting superior comb energy results compared to the fully formulated, silicone-containing conditioner.

The results are shown in Table 4. Wet comb energy values were measured with Texture Analyzer. The conditioners were applied on damaged hair 0.05 g grams per grain of bleached hair (1 hour bleached) and wet comb energies were measured after leave-on and after 1, 3 and 5 times of washing with 0.1 grams per gram hair shampoo (shampoo: 12/2 SLES/CAPB).

**Table 4**

| Example | Wet Comb Energy Leave-on (gf-mm) | Wet Comb Energy 1 Wash (gl-mm) | Wet Comb Energy 3 Washes (gf-mm) | Wet Comb Energy 5 Washes (gf-mm) |
|---|---|---|---|---|
| 2f | 1891 | 4454 | 10,396 | 10,801 |
| Commercial product | 1287 | 67,841 | 139,426 | 151,842 |

Examples 2d and 2f were compared to Example 1d (Comparative) containing 1% Polyquaternium-55, 0.65% cationic surfactant and 2.45% fatty alcohol. Polyquaternium-55 was an example of a cationic polymer. The conditioners were applied on damaged hair 0,2 grams per gram of bleached hair (1 hour bleached) and wet comb energies were measured after 1, 3 and 5 times of washing with 0.1 grams per gram hair shampoo (shampoo : 12/2 SLES/CAPB).

**Table 5**

| Example | Wet comb energy (gf-mm) after 1 x shampoo wash | Wet comb energy (gf-mm) after 3 x shampoo wash | Wet comb energy (gf-mm) after 5 x shampoo wash |
|---|---|---|---|
| 2d | 3089 | 3624 | 4265 |
| 2f | 21,264 | 13,095 | 17,782 |
| 1d (comb.) | 3561 | 95,259 | 136,105 |

From the results presented in Table 5, it can be clearly seen that the conditioning performance of Examples 2d and 2f after several washes with silicone-free conditioning performance of Examples 2d and 2f after several washes with silicone-free shampoo is superior compared to cationic polymer described in the Comparative Example 1d.

The polyAPTAC homopolymer can also be used in combination with cationic surfactants, but it has been surprisingly found that the comb energy values without additional cationic surfactant are even lower. Example 2f was compared to the formulation containing Example 6a of 1 % polyAPTAC homopolymer with Mw 500,000 Dalton and 3% fatty alcohol.

The conditioners were applied on damaged hair 0.2 grams per gram of bleached hair (1 hour bleached) and wet comb energies were measured after leave-on and after 1 and 3 times of washing with 0.1 grams per gram hair shampoo (shampoo: 12/2 SLES/CAPB). The results are presented in Table 6:

**Table 6**

|  | Wet comb energy (gf-mm) after leave-on | Wet comb energy (gf-mm) after rinse-off | Wet comb energy (gf-mm) after 1 x shampoo wash | Wet comb energy (gf-min) after 3 x shampoo wash |
|---|---|---|---|---|
| 6a | 8710 | 6979 | 11,439 | 3301 |
| 2f | 18,927 | 23,603 | 21,264 | 13,095 |

From the results presented in Table 6, it can be surprisingly seen that the conditioning performance is improved after several washes with silicone-free shampoo.

Example 3 Color Preservation (not claimed)

The improved performance after multiple washes of the conditioning systems containing the polyAPTAC homopolymer is also of value for preventing color fading in hair colors. Hair tresses were treated with a red color known for its fading issue. The hair color used was a permanent color from L'Oreal Garnier Nutrisse Crème level 3. The process used was as follows;

Opened the application bottle with developing cream, added color from the tube, closed bottle and shook until homogeneous mixture.
Started the dying process immediately onto dry hair tresses by massaging down the length of the tress for about 1 minute.
Covered the hair with aluminum foil and waited for 30 minutes.
Rinsed off with water (35 °C) by massaging the hair until the water was completely clear.
Depending on the procedure either washed with mild silicone free shampoo or treated the tress first with a conditioner.
When the hair was first washed, a conditioner was applied after washing.
When the hair was first treated with a conditioner, the hair was washed afterwards.
Let the hair dry till the next day.
Used a BYK-Gardner Spectrophotometer to measure the changes in color indexes (L*a*b).

Total hair color $\Delta$L (+lighter)
Red-green $\Delta$a (+redder, - greener)
Yellow-blue (+yellower, - bluer)

$$\text{Total difference} \quad \Delta E = \left[ (\Delta L)^2 + (\Delta a)^2 + (\Delta b)^2 \right]^{1/2}$$

Placed tress between marked lines evenly on white paper and put glass plate onto it to make the surface more even.
Measured 5 times from different locations of the tress.

The test was done with the treatment of the tresses with conditioner (1% polymer of Example 2f in cationic surfactant/fatty alcohol 0.65%/2.45%; dosage 0.2 grams/gram of hair). Right after the dyeing process, the excessive hair dye was rinsed off with water. The hair was then washed with silicone-free shampoo SLES/CAPB 12%/2% and afterwards the hair was treated with a conditioner. This washing cycle was repeated 10 times and the color difference $\Delta$E was measured after 3, 5 and 10 washing cycles. The conditioner was also compared with Fructis color resist conditioner. The test results are given in Table 7:

**Table 7**

| Example | $\Delta$E 3 washes | $\Delta$E 5 washes | $\Delta$E 10 washes |
|---|---|---|---|
| 2f | 1.43 | 2.63 | 4.25 |
| Fructis color resist conditioner | 2.76 | 3.10 | 4.57 |

From this Table, it is apparent that the Example 2f does indeed demonstrate substantial improvement in color retention after multiple washes for this color system when compared to a commercial conditioner developed especially for color protection purposes.

The test was done with the treatment of the tresses with conditioner (1% polymer of Example 2f in cationic surfactant/fatty alcohol 0.65%/2.45%; dosage 0.2 grams/gram of hair). The treatments were done right after the dyeing process when the excessive hair dye was first rinsed off with water. First conditioned tresses were afterwards washed with silicone-free shampoo SLES/CAPB 12%/2%. The conditioner was also compared with a hair without conditioner treatment and with the conditioner from Fructis dye package after 3, 5 and 10 shampoo washes. The shampoo used was 12 wt% SLES, 2 wt% CAPB. The test results are given in Table 8:

**Table 8**

| Example | ΔE 3 washes | ΔE 5 washes | ΔE 10 washes |
|---|---|---|---|
| 2f | 1.73 | 1.37 | 3.4 |
| Without conditioner | 2.32 | 2.2 | 3.89 |
| Conditioner included in the dye package | 1.2 | 2.53 | 3.99 |

From this Table, it is apparent that Example 2f does indeed demonstrate substantial improvement in color retention after multiple washes for this color system when compared to a hair without conditioner treatment and a conditioner included in the dye package. When comparing the results of Table 8 with Table 7 it can be seen that the method of application had an influence on the results. When the conditioner was applied before shampooing, the total color retention after multiple washes was improved.

Example 4 Initial and Long-Lasting Hydrophobicity (not claimed)

The polyAPTAC homopolymer of Example 2f was used by 1 wt% active with water added to 100%. A simple polymer solution comprising the polyAPTAC homopolymer was applied on bleached Caucasian hair at 0.2 g/g hair resp. and was allowed to dry for twenty four (24) hours. Then, the treated hair tress was tested for hydrophobicity by applying a water droplet onto the hair. Figures 1 and 2 are photographs of a water droplet on the surface of the hair tress treated with the polymer solution of Example 2f. Figure 2 presents a tress which was washed 5 times with silicone-free shampoo after applying the polymer solution onto the tress. From Figures 1 and 2, it can be clearly seen that the treated hair tress is very hydrophobic in nature as the water droplet on the surface of the tress exhibited a high contact angle.

From Figure 2, it can be clearly seen that the hydrophobicity was increased after multiple washes due to the interaction with the alkyl groups present in the sodium laureth ether sulfate which was used as primary surfactant.

The water droplet was observed to remain on the hair for more than 10 minutes. This droplet behaviour was evidence of the hydrophobicity of the treated tresses.

Figure 3 is a photograph of water droplets applied to bleached Caucasian hair treated with a commercial conditioner. From Figure 3, it can be clearly seen that the hair tresses treated with a commercial conditioner did not impart the same degree of hydrophobicity as was observed in the treated hair tress of Figures 1 and 2. In the hair tresses treated with a commercial conditioner, the water droplets exhibited low contact angles with the hair tress surface and were absorbed or at least immediately spread over the tress after applying the water droplet.

Example 5 Initial and Long-Lasting Hydrophobicity (not claimed)

The polyAPTAC homopolymerof Example 2f was used by 1 wt% active in conjunction with cetearyi alcohol at 2.45 wt% and with a cationic surfactant at 0.65 wt% with water added to 100%. The simple conditioner formula was then applied to hair at 0.2 g/g bleached Caucasian hair and rinsed off. After drying at RT it washed 5 times with a clarifying shampoo base (12 wt% $SLE_2S$, 2 wt% CAPB) and after the fifth time the hair tress was evaluated for retained hydrophobicity.

Figure 4 is a photograph of a water droplet on the hair tress. Figure 4 clearly demonstrates that the polyAPTAC homopolymer of Example 2f provided the treated hair tress with long lasting hydrophobicity after 5 washes with shampoo.

Example 6 Deposition of the Polymer onto the Hair Surface (not claimed)

The polyAPTAC homopolymer of Example 2f was used by 1 wt% active in conjunction with cetearyl alcohol at 2.45 wt% and with a cationic surfactant at 0.65 wt% with water added to 100%. The simple conditioner formula was then applied to hair at 0.2g/g bleached Caucasian hair and rinsed off. After drying at RT, it was washed 5x with 0.1 grams per gram of hair clarifying shampoo base (12 wt% $SLE_2S$, 2 wt% CAPB) and the surface of the treated hair tress was studied by using Scanning Electron Microscopy.

The polyAPTAC homopolymer of Example 2f was used by 1 wt% active in conjunction with cetearyl alcohol at 2.45 wt% and with a cationic surfactants at 0.65 wt% with water added to 100%, The simple conditioner formula was then applied to hair at 0.2 g/g bleached Caucasian hair and rinsed off. After drying at RT, it was treated 5 times with a washing cycle with 0.1 grams per gram of hair a clarifying shampoo base (12 wt% SLE$_2$S, 2 wt% CAPB) and afterwards 0.2 grams per gram of hair and the surface of the treated hair tress was studied by using Scanning Electron Microscopy (SEM) and Atomic Fluorescence Microscopy (AFM).

It can be seen from Figure 5 that Example 2f when used as a conditioner is able to deposit on the hair surface. A widespread filmy deposition can be clearly seen.

It can be seen from the Figures 6 and 7 that Example 2f when used as a conditioner is able to smoothen the damaged hair surface. Cuticles are aligned on the hair surface. From Figure 6 it can be seen that the elect is long-lasting. After 5 washes with clarifying shampoo the cuticles lie smoothly on the hair surface forming a homogenous layer.

The higher the contact angle the more hydrophobic is the surface. Undamaged virgin brown hair is naturally hydrophobic, but all the chemical treatments such as bleaching reduce the hydrophobicity of the hair. Table 9 demonstrates the influence of the bleaching on the contact angle.

**Table 9**

| Treatment | Contact angle, ° |
|---|---|
| Virgin brown hair | 115 |
| 1h bleaching, medium damage | 85 |
| 2.75 h bleaching | 48 |

The polyAPTAC homopolymer of Example 2f was used by 1 wt% active in conjunction with cetearyl alcohol at 2.45 wt% and with a cationic surfactant at 0.65 wt% with water added to 100%. The simple conditioner formula was then applied to hair at 0.2 g/g medium bleached Caucasian hair and rinsed off. After drying at RT, it was washed 5x with a clarifying shampoo base (12 wt% SLE$_2$S, 2 wt% CAPB). The contact angle of the treated tresses was measured before the first wash, after 2 washes and after 5 washes. The test results are given in Table 10.

**Table 10**

| Washes with clarifying shampoo | Average contact angle, ° |
|---|---|
| 0 | 96.2 |
| 2 | 95.6 |
| 5 | 93.4 |

From Table 10, it can be seen that treating medium bleached Caucasian hair tress resulted in higher contact angle values compared to the original bleached hair tress (85°). It is also shown that the hydrophobicity of the treated hair tress remains for at least 5 washes.

The long-lasting hydrophobicity is presented in Table 11. It can be seen that even after 600 s the hair tress treated with example 2f is hydrophobic.

**Table 11]**

| Example/Washes | Time of water absorption, s |
|---|---|
| Example 2f, 0 washes | 600+ |
| Example 2f, 2 washes | 440 |
| Example 2f, 5 washes | 600+ |

The polyAPTAC homopolymer of Example 2f was used by 1 wt% active in conjunction with cetearyl alcohol at 2.45 wt% and with a cationic surfactant at 0.65 wt% with water added to 100%. The simple conditioner formula was then applied to hair at 0.2 g/g medium bleached Caucasian hair and rinsed off. After drying at RT, it was washed 5x with a clarifying shampoo base (12 wt% SLE$_2$S, 2 wt% CAPB). The contact angle of the treated tresses was measured before the first wash and after 2 washes. The test results are given in Table 12.

**Table 12**

| Example | Mw, g/mol | 1x conditioner treatment, contact angle, ° | 1x conditioner treatment & 2 x wash shampoo, contact angle, ° |
|---|---|---|---|
| 2a | 100,000 | 77.8 | 83.4 |
| 2b | 100,000 | 78.9 | 82.1 |
| 2c | 200,000 | 81.7 | 90.6 |
| 2d | 300,000 | 92.3 | 94.9 |
| 2e | 1,000,000 | 89.4 | 95 |
| 2f | 500,000 | 87.9 | 94 |

From Table 12 above, it can be seen that treating medium bleached Caucasian hair tress results higher contact angle values compared to the original bleached hair tress (85°). It can be seen that there is an interaction between the polyAPTAC homopolymers and the surfactants resulting increased contact angle values after washing with clarifying shampoo. It can be also seen that the higher the molecular weight and the higher the contact angle with minimum Mw ≥ 200,000.

A trained panel of experts can evaluate the sensory properties of the treated hair tresses and the results are correlating with measured values e.g. comb energy values.

Sensory properties are essential part of consumer acceptance. With the help of the sensory evaluation the long-term properties after multiple uses can be determined.

The polymer of Example 2f was used by 1 wt% active in conjunction with cetearyl alcohol at 2.45 wt% and with a cationic surfactant at 0.65 wt% with water added to 100%, The simple conditioner formula was then applied to hair at 0.2 g/g bleached Caucasian hair and rinsed off. After drying at RT, it was treated 5 times with a washing cycle with 0.1 grams per gram of hair a clarifying shampoo base (12 wt% $SLE_2S$, 2 wt% CAPB) and afterwards 0.2 grams per gram of hair. For every evaluation, 3 tresses were prepared. The sensory properties of the tresses were evaluated after first and fifth washing cycle. A washing cycle simulated a real-life situation.

Table 13 shows the results of the sensory evaluation of the wet tresses treated with Example 2f rinse-off conditioner 0.2 g/g hair. For washing simplified silicone-free shampoo (12 wt% $SLE_2S$, 2 wt% CAPB) was used 0.1 g/g hair. The tresses were given a number based on their performance. In the ranking system used in the above evaluations, a ranking of 1 was the worst and 5 was the best value.

**Table 13**

| Parameter | After 1. Washing cycle | After 5. Washing cycle |
|---|---|---|
| Detangling | 3.5 | 4.8 |
| Combing | 3.7 | 4.8 |
| Stickiness | 3.8 | 4.3 |
| Slipperiness | 4 | 4.2 |
| Smoothness | 4.2 | 4 |
| Coatedness | 4.2 | 4.5 |

From Table 13, it can be seen that the results after the first washing cycle are already good. After 5 washing cycles, the sensory properties have been even improved being excellent. The tresses performed well in every category with no negative side-effects being observed.

Example 7 Combinations of PolyAPTAC Homopolymers with Various Surfactants (not claimed)

The polyAPTAC homopolymer of Example 2f was tested at 1 wt% in 2.5 % fatty alcohol and 0.7 % cationic surfactants. The cationic surfactants used for this Example were cetrimonium chloride (Example 7a), behentrimonium chloride (Example 7b) and stearamidopropyl dimethylamine (Example 7c). The polyAPTAC homopolymer/surfactant_conditioner composition was applied on damaged hair (0.2 grams per gram of bleached hair, 1 hour bleached) and wet comb energies were measured with Texture Analyzer after rinse with water and after 1, 3 and 5 times of washing with 0.1 grams per gram hair shampoo (shampoo: 12/2 SLES/CAPB). The test results are provided in Table 14:

**Table 14**

| Example | Wet Comb rinse-off conditioner (gf-mm) | Wet Comb 1 wash (gf-mm) | Wet Comb 3 washes (gf-mm) | Wet Comb 5 washes (gf-mm) |
|---|---|---|---|---|
| 7a | 4386 | 1628 | 1838 | 1869 |
| 7b | 4108 | 1878 | 1683 | 1877 |
| 7c | 2263 | 3089 | 3624 | 4265 |

From the above results presented in Table 14, it is evident that a PolyAPTAC homopolymer can be combined with different cationic surfactants resulting very low comb energy values.

Example 8 Hydrophobically Modified PolyAPTAC (MW = 1,000,000)

The polyAPTAC was hydrophobically modified using different levels of hydrophobic co-monomers. The levels of the hydrophobic co-monomers used in this Example were 1.5 % by weight (Example 8a), 3% by weight (Example 8b) and 5% by weight (Example 8c). Stearyl acrylate was used as hydrophobic co-monomer in each of the polyAPTAC modified. The molecular weights of these examples were 1,000,000 Dalton. The resultant hydrophobically modified polymers were tested as a 1 wt% in 2.45 % fatty alcohol and 0.65 % cationic surfactants. As a reference, a conditioner chassis without additional cationic polymer was tested and compared to the conditioner samples containing the hydrophobically modified polyAPTAC All of the conditioner samples were applied on damaged hair (0.2 grams per gram of bleached hair, 1 hour bleached) and the wet comb energies of the conditioned hair samples were measured with Texture Analyzer after the rinse with water and after 1, 3 and 5 times of washing with 0.1 grams per gram hair shampoo (shampoo: 12/2 SLES/CAPB). The test results are provided in Table 15:

**Table 15**

| Example | Wet Comb rinse-off conditioner (gf-mm) | Wet Comb 1 wash (gf-mm) | Wet Comb 3 washes (gf-mm) | Wet Comb 5 washes (gf-mm) |
|---|---|---|---|---|
| 8a | 4482 | 4980 | 3620 | 3543 |
| 8b | 3542 | 5146 | 4243 | 4927 |
| 8c | 4696 | 4830 | 5089 | 6213 |
| Reference | 1348 | 72,978 | 110,254 | 116,495 |

From the above results presented in Table 15, it is evident that a hydrophobically modified polymer results very low comb energy values showing also excellent long lasting benefits.

Example 9 Hydrophobically modified polyAPTAC (MW = 500,000)

The hydrophobically modified polyAPTAC using different levels of hydrophobic co-monomers were also shown in this example. Stearyl acrylate was used as hydrophobic co-monomer. The levels of stearyl acrylate used were 1.5 % by weight (Example 9a), 3% by weight (Example 9b), 5% by weight (Example 9c) and 10% by weight (Example 9d).
The resultant hydrophobically modified polymers were tested as a 1 wt% in 3 % fatty alcohol. As a reference, a polyAPTAC homopolymer in fatty alcohol was used, All of these polymers had molecular weights of about 500,000 Dalton. Additionally, a conditioner chassis without additional cationic polymer was tested and compared to the conditioner example containing the polyAPTAC homopolymer and hydrophobically modified polyAPTAC. All of the conditioner samples were applied on damaged hair (0.2 grams per gram of bleached hair, 1 hour bleached) and the wet comb energies of the conditioned hair samples were measured with Texture Analyzer after the rinse with water and after 1, 3 and 5 times of washing with 0.1 grams per gram hair shampoo (shampoo: 12/2 SLES/CAPB). The test results are provided in Table 16:

**Table 16**

| Example | Wet Comb rinse-off conditioner (gf-mm) | Wet Comb 1 wash (gf-mm) | Wet Comb 3 washes (gf-mm) | Wet Comb 5 washes (gf-mm) |
|---|---|---|---|---|
| 9a | 3324 | 2192 | 2494 | 3379 |
| 9c | 3791 | 2702 | 2421 | 2749 |
| 9d | 2642 | 2980 | 2717 | 3257 |
| Reference | 6979 | 11,439 | 3301 | 4141 |
| Conditioner chassis | 1348 | 72,978 | 110,254 | 116,495 |

From the above results presented in Table 16, it is evident that a hydrophobically modified polymer results very low comb energy values showing also excellent long lasting benefits. It is also evident that hydrophobic modification is reducing the initial wet comb energy values compared to the homopolymer. The higher the level of hydrophobic modification is, the lower the initial wet comb energy values are,

PEG-18 Behenyl Methacrylate (BEM) was used as a hydrophobic co-monomer as levels 2.5% by weight (Example 9e), 5% by weight (Example 9f) and 10% by weight (Example 9g). The resultant hydrophobically modified polymers were tested as a 1 wt% in 2.45 % fatty alcohol 0.65 wt% cationic surfactant. As a reference, a polyAPTAC homopolymer was tested as 1 wt% in 2.45% fatty alcohol and 0.65 wt% cationic surfactant. All of these polymers had molecular weights of about 500,000 Dalton. Additionally, a conditioner chassis without additional cationic polymer was tested and compared to the conditioner examples containing the polyAPTAC homopolymer and the hydrophobically modified polyAPTAC. All of the conditioners samples were applied on damaged hair (0.2 grams per gram of bleached hair, 1 hour bleached) and the wet comb energies of the conditioned hair samples were measured with Texture Analyzer after the rinse with water and after 1, 3 and 5 times of washing with 0.1 grams per gram hair shampoo (shampoo: 12/2 SLES/CAPB). The test results are provided in Table 17:

**Table 17**

| Example | Wet Comb rinse-off conditioner (gf-mm) | Wet Comb 1 wash (gf-mm) | Wet Comb 3 washes (gf-mm) | Wet Comb 5 washes (gf-mm) |
|---|---|---|---|---|
| 9e | 10926 | 2131 | 2344 | 2382 |
| 9f | 8565 | 2798 | 3221 | 3490 |
| 9g | 7640 | 2255 | 1789 | 2331 |
| Reference | 12148 | 2877 | 3951 | 3995 |
| Conditioner chassis | 1348 | 72,978 | 110,254 | 116,495 |

From the above results presented in Table 17, it is evident that a hydrophobically modified polymer results very low comb energy values showing also excellent long lasting benefits. It is also evident that hydrophobic modification is reducing the initial wet comb energy values compared to the homopolymer. The higher the level of hydrophobic modification is, the lower the initial wet comb energy values are.

Example 10 Contact Angle - Hydrophobically Modified PolyAPTAC (MW = 500,000)

Table 18 shows the results demonstrating the influence of the bleaching on the contact angle.

**Table 18**

| Treatment | Contact angle, ° |
|---|---|
| Virgin brown hair | 115 |
| 1h bleaching, medium damage | 85 |
| 2.75 h bleaching | 48 |

The hydrophobically modified polyAPTAC of Examples 9a - 9d were used by 1 wt% active in conjunction with cetearyl alcohol at 3 wt% and with water added to 100%. A polyAPTAC homopolymer (MW 500,000) was used as a reference. The simple conditioner formula was then applied to hair at 0.2 g/g medium bleached Caucasian hair and rinsed off. The test results are given in Table 19.

**Table 19**

| Example | Hydrophobic monomer | wt% | Average contact angle, ° |
|---------|---------------------|-----|--------------------------|
| 9a | Stearyl Acrylate | 1.5 | 100.9 |
| 9b | Stearyl Acrylate | 3 | 107 |
| 9c | Stearyl Acrylate | 5 | 106.1 |
| 9d | Stearyl Acrylate | 10 | 106.4 |
| Reference | PolyAPTAC homopolymer | - | 96.2 |

From Table 19, it can be seen that treating medium bleached Caucasian hair tress resulted in higher contact angle values compared to the original bleached hair tress (85°). It is also shown that the hydrophobic modification increases the initial contact angle value.

The hydrophobically modified polyAPTAC polymer of Example 8a was used by 1 wt% active in conjunction with cetearyl alcohol at 2.45 wt% and with a cationic surfactant at 0.65 wt% with water added to 100 wt%. The simple conditioner formula was then applied to hair at 0.2 g/g medium bleached Caucasian hair and rinsed off. After drying at RT, it was washed 2x with a clarifying shampoo base (12 wt% SLE$_2$S, 2 wt% CAPB). The contact angle of the treated tresses was measured before the first wash and after 2 washes. The formula was compared to the reference consisting of fatty alcohol in combination with cationic surfactant and to the formula containing polyAPTAC homopolymer (MW 1,000,000). The test results are given in Table 20.

**Table 20**

| Example | Initial contact angle | Contact angle after 2 washes with a non-conditioning shampoo |
|---------|----------------------|-------------------------------------------------------------|
| Reference | 85.5 | 74.4 |
| Homopolymer polyAPTAC | 89.7 | 96.6 |
| 8a | 89.5 | 101.5 |

From the table above it can be seen that the hydrophobic modification presented in Example 8a is improving the hydrophobicity of the hair surface even after several washes with non-conditioning shampoo compared to the reference and the formula containing polyAPTAC homopolymer.

Example 11 Emulsion stabilisation

A typical conditioner formulation is an oil-in-water (o/w) emulsion consisting of primary emulsifier, co-emulsifier such as fatty alcohols and cationic surfactants such as cetrimonium chloride or behentrimonium chloride. A polyAPTAC homopolymer doesn't have any emulsifying properties and for that reason cationic surfactants cannot be fully replaced by polyAPTAC homopolymer. Hydrophobic modification of polyAPTAC, which is presented in the Examples 9a to 9d improved the emulsion stabilization properties of the cationic polymer. Figure 8 is presented typical structure of the conditioner consisting of 1 wt% polyAPTAC homopolymer (MW 1,000,000) in combination with 3 wt% fatty alcohol. Figure 9 is presented the hydrophobically modified polyAPTAC of Example 9c in combination with 3 % fatty alcohol. It can be clearly seen that hydrophobic modification has improved the emulsion stabilization properties.

Example 12 Surfactant based applications

Hydrophobic modification makes the polyAPTAC better compatible for surfactant based applications than polyAPTAC homopolymer (MW 1,000,000 as Reference). Due to the high cationic charge the homopolymer tends to form complexes with anionic surfactants. Table 21 below shows wet comb energy results of 0.2 wt% of Example 9e in combination with 12 wt% SLES 2EO and 2 wt% CAPB. The results are compared to Commercial shampoo without silicones.

**Table 21**

| Example | Wet comb energy after 1 wash, g*mm | Wet comb energy after 3 washes with non-conditioning shampoo, g*mm |
|---|---|---|
| Example 9e | 37,000 | 10,000 |
| Reference | na | na |
| L'Oreal Garnier Fructis shampoo | 62.919 | 54.200 |

From the Table above it can be seen that Example 9e outperforms the commercial product. The performance is even sustained after 3 washes.

**Claims**

1. A conditioning composition additive for providing immediate and prolonged benefit to a keratin surface comprising:

   a) a hydrophobically modified poly(acrylamido-N-propyltrimethylammonium chloride) (polyAPTAC); and
   b) water,

   wherein the hydrophobically modified polyAPTAC is present in an amount of from 0.1 wt% to 20 wt% of the total weight of the conditioning composition additive and has a cationic charge density in the range of 1 to 8 meq/g; and wherein the hydrophobically modified polyAPTAC comprises 1.5% to 15% by weight of a hydrophobic co-monomer selected from the group consisting of alkyl acrylate, alkyl methacrylate, alkyl ethoxylate (meth)acrylate, ethylene glycol behenyl ether methacrylate, and alkyl aryl (meth)acrylate.

2. The conditioning composition additive of claim 1, wherein the hydrophobic co-monomer comprises stearyl acrylate.

3. The conditioning composition additive of claim 1, wherein the hydrophobically modified polyAPTAC has a molecular weight in the range of from 100,000 to 1,000,000 g/mol; preferably wherein the hydrophobically modified polyAPTAC has a molecular weight in the range of from 200,000 to 500,000 g/mol.

4. The conditioning composition additive of claim 1, further comprising a substantive polymer selected from the group consisting of polysaccharides and synthetic cationic polymers.

5. The conditioning composition additive of claim 4, wherein the polysaccharide is a cellulose derivative; preferably hydroxyethyl cellulose; more preferably hydrophobically modified hydroxyethyl cellulose.

6. The conditioning composition additive of claim 4, wherein the polysaccharide is a polygalactomannan; preferably guar; more preferably hydrophobically modified guar.

7. The conditioning composition additive of claim 1, wherein the hydrophobically modified polyAPTAC has a cationic charge density in the range of 4 to 6 meq/g.

8. The conditioning composition additive of claim 1, wherein the hydrophobically modified polyAPTAC is present in an amount of from 0.25 wt % to 5 wt% of the total weight of the conditioning composition additive.

9. A conditioning composition comprising

   a) the conditioning composition additive according to claim 1; and
   b) a personal care active ingredient.

10. The conditioning composition of claim 9, wherein the conditioning composition comprises

    - a hair care product; or

- a skin care product.

11. A method for providing immediate and prolonged benefit to a keratin surface comprising:

applying a conditioning composition to the surface,
wherein the conditioning composition comprises a conditioning composition additive according to claim 1 and a personal care active ingredient.

**Patentansprüche**

1. Pflegespülungszusammensetzungsadditiv, mit der einer Keratinoberfläche ein sofortiger und verlängerter Nutzen verliehen wird, umfassend:

a) ein hydrophob modifiziertes Poly(acrylamido-N-propyltrimethylammoniumchlorid) (PolyAPTAC); und
b) Wasser,

wobei das hydrophob modifizierte PolyAPTAC in einer Menge von 0,1 Gew.-% bis 20 Gew.-% des Gesamtgewichts des Pflegespülungszusammensetzungsadditivs vorliegt und eine kationische Ladungsdichte im Bereich von 1 bis 8 mÄq/g aufweist; und
wobei das hydrophob modifizierte PolyAPTAC 1,5% bis 15 Gew.-% eines hydrophoben Comonomers umfasst, ausgewählt aus der Gruppe, bestehend aus Alkylacrylat, Alkylmethacrylat, Alkylethoxylat(meth)acrylat, Ethylenglycolbehenylethermethacrylat und Alkylaryl(meth)-acrylat.

2. Pflegespülungszusammensetzungsadditiv nach Anspruch 1, wobei das hydrophobe Comonomer Stearylacrylat umfasst.

3. Pflegespülungszusammensetzungsadditiv nach Anspruch 1, wobei das hydrophob modifizierte PolyAPTAC ein Molekulargewicht im Bereich von 100000 bis 1000000 g/mol aufweist; wobei das hydrophob modifizierte PolyAPTAC vorzugsweise ein Molekulargewicht im Bereich von 200000 bis 500000 g/mol aufweist.

4. Pflegespülungszusammensetzungsadditiv nach Anspruch 1, zudem umfassend ein wesentliches Polymer, ausgewählt aus der Gruppe, bestehend aus Polysacchariden und synthetischen kationischen Polymeren.

5. Pflegespülungszusammensetzungsadditiv nach Anspruch 4, wobei das Polysaccharid ein Cellulosederivat; vorzugsweise Hydroxyethylcellulose; stärker bevorzugt hydrophob modifizierte Hydroxyethylcelulose ist.

6. Pflegespülungszusammensetzungsadditiv nach Anspruch 4, wobei das Polysaccharid ein Polygalactomannan; vorzugsweise Guaran; stärker bevorzugt hydrophob modifiziertes Guaran ist.

7. Pflegespülungszusammensetzungsadditiv nach Anspruch 1, wobei das hydrophob modifizierte PolyAPTAC eine kationische Ladungsdichte im Bereich von 4 bis 6 mÄq/g aufweist.

8. Pflegespülungszusammensetzungsadditiv nach Anspruch 1, wobei das hydrophob modifizierte PolyAPTAC in einer Menge von 0,25 Gew.-% bis 5 Gew.-% des Gesamtgewichts des Pflegespülungszusammensetzungsadditivs vorliegt.

9. Pflegespülungszusammensetzung, umfassend

a) das Pflegespülungszusammensetzungsadditiv nach Anspruch 1; und
b) einen Körperpflegewirkstoff.

10. Pflegespülungszusammensetzung nach Anspruch 9, wobei die Pflegespülungszusammensetzung umfasst:

- ein Haarpflegeprodukt; oder
- ein Hautpflegeprodukt.

11. Verfahren, mit dem einer Keratinoberfläche ein sofortiger und verlängerter Nutzen verliehen wird, umfassend:

Aufbringen einer Pflegespülungszusammensetzung auf die Oberfläche,
wobei die Pflegespülungszusammensetzung ein Pflegespülungszusammensetzungsadditiv nach Anspruch 1
und einen Körperpflegewirkstoff umfasst.

**Revendications**

1.  Additif pour composition de conditionnement, destiné à apporter un bénéfice immédiat et prolongé à une surface kératinique, comprenant :

    a) un poly(chlorure d'acrylamido-N-propyltriméthyl-ammonium) modifié de manière hydrophobe (polyAPTAC) ; et
    b) de l'eau ;

    où le polyAPTAC modifié de manière hydrophobe est présent selon une quantité allant de 0,1% en poids à 20% en poids du poids total de l'additif pour composition de conditionnement et possède une densité de charge cationique dans la plage allant de 1 à 8 méq/g ; et
    où le polyAPTAC modifié de manière hydrophobe comprend de 1,5% à 15% en poids d'un co-monomère hydrophobe choisi dans le groupe constitué par un acrylate d'alkyle, un méthacrylate d'alkyle, un (méth)acrylate d'alkyle éthoxylé, un méthacrylate d'éther béhénylique d'éthylène glycol, et un (méth)acrylate d'alkylaryle.

2.  Additif pour composition de conditionnement selon la revendication 1, dans lequel le co-monomère hydrophobe comprend un acrylate de stéaryle.

3.  Additif pour composition de conditionnement selon la revendication 1, dans lequel le polyAPTAC modifié de manière hydrophobe possède un poids moléculaire dans la plage allant de 100 000 à 1 000 000 g/mol ; préférablement où le polyAPTAC modifié de manière hydrophobe possède un poids moléculaire dans la plage allant de 200 000 à 500 000 g/mol.

4.  Additif pour composition de conditionnement selon la revendication 1, comprenant en outre un polymère substantif choisi dans le groupe constitué par les polysaccharides et les polymères cationiques synthétiques.

5.  Additif pour composition de conditionnement selon la revendication 4, dans lequel le polysaccharide est un dérivé de cellulose ; préférablement l'hydroxyéthylcellulose ; plus préférablement l'hydroxyéthylcellulose modifiée de manière hydrophobe.

6.  Additif pour composition de conditionnement selon la revendication 4, dans lequel le polysaccharide est un polygalactomannane ; préférablement la gomme de guar ; plus préférablement la gomme de guar modifiée de manière hydrophobe.

7.  Additif pour composition de conditionnement selon la revendication 1, dans lequel le polyAPTAC modifié de manière hydrophobe possède une densité de charge cationique dans la plage allant de 4 à 6 méq/g.

8.  Additif pour composition de conditionnement selon la revendication 1, dans lequel le polyAPTAC modifié de manière hydrophobe est présent selon une quantité allant de 0,25% en poids à 5% en poids du poids total de l'additif pour composition de conditionnement.

9.  Composition de conditionnement, comprenant

    a) l'additif pour composition de conditionnement selon la revendication 1 ; et
    b) un ingrédient actif de soin personnel.

10. Composition de conditionnement selon la revendication 9, où la composition de conditionnement comprend

    - un produit de soin des cheveux ; ou
    - un produit de soin de la peau.

11. Méthode destinée à apporter un bénéfice immédiat et prolongé à une surface kératinique, comprenant

l'application d'une composition de conditionnement à la surface,
où la composition de conditionnement comprend un additif pour composition de conditionnement selon la revendication 1 et un ingrédient actif de soin personnel.

Figure 1

Figure 2

Figure 3

Figure 4

**Figure 5**

**Figure 6**

Figure 7

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03101410 A
- US 20120076747 A
- WO 9401076 A
- US 6489286 B
- EP 1064927 A
- DE 102004045253
- EP 1021471 B1
- DE 3929973